# EUROPEAN PATENT APPLICATION

(11) **EP 4 349 962 A1**
(43) Date of publication of application: **10.04.2024**
(21) Application number: 22862712.1
(22) Date of filing: 11.05.2022
(51) Int. Cl.: C12N 1/20, C12N 1/02, C12J 1/04, C12R 1/225

(54) **STRAIN HSCY 2073, AND ISOLATION AND SCREENING THEREFOR AND USE THEREOF IN IMPROVING FLAVOR AND QUALITY OF VINEGAR**

(30) Priority: 30.08.2021 CN 202111001737
(71) Applicant: Jiangsu Hengshun Vinegar-Industry Co., Ltd, Zhenjiang, Jiangsu 212028 (CN)
(72) Inventor: LI, Xin, Zhenjiang, Jiangsu 212028 (CN); ZHANG, Junhong, Zhenjiang, Jiangsu 212028 (CN); XIONG, Feng, Zhenjiang, Jiangsu 212028 (CN); CAI, Panpan, Zhenjiang, Jiangsu 212028 (CN); ZHANG, Bao, Zhenjiang, Jiangsu 212028 (CN); ZHU, Jie, Zhenjiang, Jiangsu 212028 (CN); DENG, Huixin, Zhenjiang, Jiangsu 212028 (CN); CUI, Pengjing, Zhenjiang, Jiangsu 212028 (CN); LIU, Yang, Zhenjiang, Jiangsu 212028 (CN); QIAO, Xin, Zhenjiang, Jiangsu 212028 (CN)
(74) Representative: Murgitroyd & Company
(86) International application number: PCT/CN2022/092170
(87) International publication number: WO 2023/029569

(57) **Abstract**

Disclosed are bacterial strain HSCY 2073, isolation and screening thereof, and application thereof in improvement on flavor quality of vinegar. Bacterial strain HSCY 2073 is identified as *Lactobacillus sp.* and is deposited in China General Microbiological Culture Collection Center, where deposit date is 21 November 2019, and deposit number is CGMCC No. 18982. Bacterial strain HSCY 2073 in the present disclosure is *Lactobacillus sp.* that has strong acetic acid and ethanol tolerance and excellent effect of producing non-volatile acid, and can produce high contents of 5 aroma substances including acetaldehyde, ethyl acetate, isobutyl acetate, isoamyl acetate and ethyl 3-methylvalerate that have fruit aromas. The lactobacillus can adapt to high-acid, high-alcohol and oligotrophic harsh environments, etc. of vinegar brewing, and has strong ability of high acid tolerance.

## Description

### TECHNICAL FIELD

The present disclosure relates to the field of traditional vinegar brewing, and particularly relates to bacterial strain HSCY 2073, isolation and screening thereof, and application thereof in improvement on flavor quality of vinegar.

### BACKGROUND

Vinegar is a kind of significant condiment. A product blended with glacial acetic acid cannot be claimed as vinegar, and only a produce subjected to vinegar brewing can be called vinegar. China is a production and consumption power of vinegar. Traditional vinegar brewed through a solid-state method, such as Zhenjiang aromatic vinegar and Shanxi aged vinegar, is a protected variety of geographical indications in China and a typical traditional fermented food in China. Unique flavor quality is a vital feature of the vinegar. A unique "solid-state layered fermentation process" of Zhenjiang aromatic vinegar has been listed in the first batch of intangible cultural heritage in China. Traditionally, the vinegar is brewed through open fermentation without sterilization of raw materials and auxiliary materials. There are extremely rich microbial species in a brewing process. Xu Zhenghong and other scholars analyzed microbial structures in a solid-state vinegar culture of Zhenjiang aromatic vinegar and found that hundreds of microorganisms are involved in fermentation in this process.

Lactobacillus refers to a type of microorganisms that can ferment carbohydrates to produce lactic acid. The lactobacillus is commonly found in a vinegar fermentation process. Especially in a traditional solid-state vinegar brewing process, a proportion of the lactobacillus can reach 60% or above, which has a vital impact on product quality. It is of significance for the vinegar brewing industry to research different types of lactobacillus and their application. Consumers have increasingly urgent demands for high-quality vinegar as living standards are improved. It is extremely urgent for the vinegar industry to brew vinegar having high flavor quality. The entire industry is in urgent need of transformation and upgrading.

At present, lactobacillus commonly used in vinegar brewing can produce acid well, but overall flavor is poor. Thus, it is a vital technical problem in the art to research and develop lactobacillus having strong acetic acid tolerance, desirable non-volatile acid production effect and excellent aroma substance production ability, which is applied in vinegar brewing as a fermentation bacterial strain to improve overall flavor quality.

In a vinegar brewing process, conventional lactobacillus is mainly used to increase non-volatile acid, saccharomycetes is mainly used to increase aroma substances, and a small number of acetic acid bacteria produce some esters besides acetic acid. However, little of single lactobacillus that can adapt to high-acid, high-alcohol and oligotrophic harsh environments, etc. of vinegar brewing is reported. That is, the lactobacillus that can produce non-volatile acid to improve softness of taste of the vinegar, can increase aroma substances such as aldehydes and esters having excellent aromas, and finally can greatly improve overall flavor quality of vinegar products is rarely reported. In addition, conventional lactobacillus has poor high acid tolerance, and is difficult to grow and metabolize in a vinegar liquid system having acidity exceeding 8 g/100 mL.

Therefore, overall flavor quality of traditional vinegar is improved by innovating isolation and culture methods and relying on a new lactobacillus bacterial strain that can adapt to harsh environments of vinegar brewing, which has a desirable application prospect.

### SUMMARY

An objective of the present disclosure provides bacterial strain HSCY 2073, which is identified as *Lactobacillus sp*., so as to solve the problems in the prior art. Bacterial strain HSCY 2073 in the present disclosure is *Lactobacillus sp.* that has strong acetic acid and ethanol tolerance and excellent effect of producing non-volatile acid, and can produce high contents of 5 aroma substances including acetaldehyde, ethyl acetate, isobutyl acetate, isoamyl acetate and ethyl 3-methylvalerate that have fruit aromas. The lactobacillus can adapt to high-acid, high-alcohol and oligotrophic harsh environments, etc. of vinegar brewing, and has strong ability of high acid tolerance. The lactobacillus can produce non-volatile acid to improve softness of taste of vinegar, can increase aroma substances such as aldehydes and esters that have excellent aromas, and finally can greatly improve overall flavor quality of vinegar products.

Another objective of the present disclosure is to provide isolation and screening of bacterial strain HSCY 2073 and application of bacterial strain HSCY 2073 in traditional vinegar brewing.

A technical solution is as follows: in order to realize the above objective, bacterial strain HSCY 2073 as described in the present disclosure is identified as *Lactobacillus sp.* and deposited in China General Microbiological Culture Collection Center. Deposit date is 21 November 2019. Deposit number is CGMCC No. 18982. Deposit address is Institute of Microbiology, Chinese Academy of Sciences, Beijing, China. Postal code is 100101. The bacterial strain is separated from collected solid-state vinegar cultures after fermentation of different batches of Zhenjiang aromatic vinegar, Shanxi aged vinegar, Sichuan bran vinegar and vinegar of other different manufacturers by an inventor. Main biological features of bacterial strain HSCY 2073 are as follows: the bacterial strain is a gram-positive bacterium and anaerobic, and after culturing is carried out on an improved MRS culture medium for 7 days, a bacterial colony is round, an edge is neat, and a color is milky white.

Isolation and screening of bacterial strain HSCY 2073 in the present disclosure includes steps as follows:
(1) collecting solid-state vinegar cultures after fermentation, and carrying out sealing, standing, enriching and culturing;
(2) diluting an enriched sample, and coating, with the enriched sample, an improved solid de man, rogosa and sharpe (MRS) plate for culture;
(3) selecting a bacterial colony having a fast growth speed, large colony area and fruit aroma, and carrying out streaking and purifying on the improved solid MRS plate through a streaking method; and
(4) inoculating a purified bacterial strain into an improved liquid MRS culture medium, carrying out standing and culturing in a sealed culture box, measuring a yield of non-volatile acid, screening out a bacterial strain having a high yield of non-volatile acid, carrying out repetition, and obtaining bacterial strain HSCY 2073 through screening.

Application of bacterial strain HSCY 2073 in the present disclosure in improvement on flavor quality of vinegar is as follows:
Bacterial strain HSCY 2073 increases contents of non-volatile acid and aroma substances in the vinegar.
Bacterial strain HSCY 2073 increases contents of flavor substances including non-volatile acid, acetaldehyde, ethyl acetate, isobutyl acetate, isoamyl acetate and ethyl 3-methylvalerate in the vinegar.

Application of bacterial strain HSCY 2073 in the present disclosure in aromatic vinegar brewing is as follows:
Bacterial strain HSCY 2073 and a starter solid-state vinegar culture of aromatic vinegar are combined for enhanced application in aromatic vinegar brewing.

Enhanced application of bacterial strain HSCY 2073 in the present disclosure in aged vinegar brewing is as follows:
Bacterial strain HSCY 2073 and a solid-state vinegar culture of aged vinegar are combined for enhanced application in aged vinegar brewing.
Beneficial effects are as follows: compared with the prior art, the present disclosure has advantages as follows:
   (1) Bacterial strain HSCY 2073 is extremely suitable for a harsh brewing environment of traditional solid-state vinegar. The bacterial strain has strong acetic acid tolerance, can tolerate total acid of 8.0 g/100 mL to a maximum extent, and can tolerate ethanol of 13%(v/v). The bacterial strain can metabolize at 15°C.
   (2) Through enhanced application of only single bacterial strain HSCY 2073 in traditional solid-state vinegar brewing, contents of flavor substances including non-volatile acid, acetaldehyde, ethyl acetate, isobutyl acetate, isoamyl acetate and ethyl 3-methylvalerate in products can be significantly increased, overall flavor quality of the products can be significantly improved and a yield can be increased.
   (3) Bacterial strain HSCY 2073 is obvious in industrial application effect in vinegar brewing, convenient in operation and low in cost, and can obviously increase contents of non-volatile acid and aroma substances in vinegar. By using the bacterial strain in Zhenjiang aromatic vinegar brewing, a content of total acid in a solid-state vinegar culture can be increased by 4.71%, a content of non-volatile acid can be increased by 24.86%, and a product yield can be increased by 5.14%. In cases of the five flavor substances having fruit aromas, a content of acetaldehyde can be increased by 9.05%, a content of ethyl acetate can be increased by 25.83%, a content of isobutyl acetate can be increased by 1.51%, a content of isoamyl acetate can be increased by 7.39%, and a content of ethyl 3-methylvalerate can be increased by 34.27%. Taste and aromas of products are significantly improved, and overall flavor quality is high. Moreover, flavor quality of Shanxi aged vinegar can also be effectively enhanced by using the bacterial strain in Shanxi aged vinegar brewing.

### BRIEF DESCRIPTION OF THE DRAWINGS

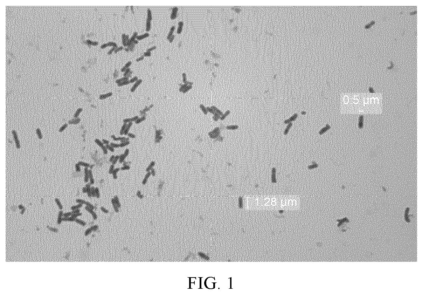

FIG. 1 shows a photograph of a shape of bacterial strain HSCY 2073 according to the present disclosure.

### DETAILED DESCRIPTION OF THE EMBODIMENTS

The present disclosure will be further described below in combination with accompanying drawings and examples.

Raw materials and reagents in the present disclosure are commercially available unless otherwise specified.

Bacterial strain *Acetilactobacillus jinshanensis* HSLZ-75^{T} has a bacterial strain deposit number of CICC 6269 and is provided by Jiangsu Hengshun Vinegar-industry Co., Ltd.

Improved solid de man, rogosa and sharpe (MRS) culture medium in examples: 10.0 g of tryptone, 10.0 g of a beef extract, 5 g of a yeast extract, 150 g of a malt extract, 20.0 g of glucose, 2.0 g of diammonium hydrogen citrate, 5.0 g of sodium acetate, 2.0 g of potassium phosphate dibasic, 0.19 g of manganese sulfate monohydrate, 0.58 g of magnesium sulfate heptahydrate, 1.0 mL of tween 80, 1.0 g of L-cysteine, 20.0 g of agar and 1 L of distilled water were provided, a temperature was 115°C, sterilization was carried out for 25 min, and cooling was carried out to 55 °C or above, and acetic acid was added for adjustment to achieve total acid of 7 g/100 mL.

Improved solid MRS plate in examples: 15 mL - 20 mL of the above sterile improved solid MRS culture medium was poured into the plate. an improved solid MRS plate was obtained after condensation.

Improved liquid MRS culture medium in examples: 10.0 g of tryptone, 10.0 g of a beef extract, 5 g of a yeast extract, 150 g of a malt extract, 20.0 g of glucose, 2.0 g of diammonium hydrogen citrate, 5.0 g of sodium acetate, 2.0 g of potassium phosphate dibasic, 0.19 g of manganese sulfate monohydrate, 0.58 g of magnesium sulfate heptahydrate, 1.0 mL of tween 80, 1 g of L-cysteine and 1 L of distilled water were provided, a temperature was 115 °C, sterilization was carried out for 25 min, and acetic acid was added for adjustment to achieve total acid of 7 g/100 mL.

Measurement methods for indexes in examples: contents of total acid and non-volatile acid are measured according to a method in GB18187-2000, the total acid is computed according to acetic acid, and the non-volatile acid is computed according to lactic acid. Aroma substances such as acetaldehyde, ethyl acetate, isobutyl acetate, isoamyl acetate and ethyl 3-methylvalerate are determined according to a method in document *Analysis of Volatile Components and Study on Age Discrimination of Zhenjiang Aromatic Vinegar* of scholar Sun Zongbao.

A reference document of a traditional Zhenjiang aromatic vinegar brewing process is as follows: Shen Zhiyuan, Yu Yongjian, Zhu Shenghu, et al. Research on Key Techniques of Non-material Cultural Heritage of Zhenjiang Vinegar Brewing Techniques[J]. China Condiment, 2016(10).

A reference document of a traditional Shanxi aged vinegar brewing process is as follows: Yan Jingzong; Yandan. Brewing technique of traditional Shanxi aged vinegar[J]. China Brewing, 2004, 2004(007):30-32.

In enhanced application of traditional Zhenjiang aromatic vinegar brewing, 26 kg of a starter solid-state vinegar culture was added to an upper portion of a solid-state vinegar culture in each cylinder. A reference document is as follows: Li Xin, Yu Yongjian, Zhu Shenghu, et al. Optimization of Starter Solid-State Vinegar Culture in Zhenjiang aromatic vinegar brewing[J]. Food and Fermentation Industries, 2017(03):115-119.

### Example 1

### Isolation and identification of bacterial strain HSCY 2073

### (1) Enrichment and culture of bacterial strains

Solid-state vinegar cultures after fermentation of different batches of Zhenjiang aromatic vinegar. Shanxi aged vinegar, Sichuan bran vinegar and vinegar of other different manufacturers were collected. 250 g of each sample was weighed and added into 250 mL of a sterilized glucose aqueous solution, where a glucose content was 2%(w/w). Sealing was carried out. Standing, enriching and culturing were carried out in a culture box at 35 °C for 14 days.

### (2) Isolation of bacterial strains

Specifically, 25 ml of each enriched sample mentioned above was added into 225 ml of a sterile physiological saline solution. Uniform shaking was carried out. 1 ml of each sample was added into 9 ml of a sterile physiological saline solution. Uniform mixing was carried out on a vortex mixer. 1 ml of a diluted solution was added into 9 ml of a physiological saline solution, and so on. Dilution was carried out for 7 times. An improved solid MRS plate was coated with a stock solution and all diluted samples in sequence. Culturing was carried out at 35°C for 15 days.

### (3) Primary screening and purification of bacterial strains

Observation was carried out every day. A bacterial colony having a fast growth speed, large bacterial colony area and fruit aroma was selected. An expected bacterial colony was selected. Streaking and purifying were carried out on the improved solid MRS plate 2 times through a streaking method.

### (4) Secondary screening of bacterial strains

The bacterial strains were purified for 3 times through the plate streaking method, and then cultured to logarithmic phases by using a liquid culture medium. The bacterial strains were inoculated into an improved liquid MRS culture medium according to an inoculation amount of 5%(v/v). Sealing was carried out. Standing and culturing were carried out in a culture box at 35 °C for 15 days - 20 days. A yield of non-volatile acid was measured. A bacterial strain having a high yield of non-volatile acid was screened out.

Steps (1) to (4) were repeated. A bacterial strain that is anaerobic and acetic acid resistant, and produces non-volatile acid, acetaldehyde, ethyl acetate, isobutyl acetate, isoamyl acetate, ethyl 3-methylvalerate and other fruit aroma substances was obtained through screening. The bacterial strain was named bacterial strain HSCY 2073. A shape of bacterial strain HSCY 2073 was as shown in FIG. 1.

### (5) Identification of bacterial strains

Genomic deoxyribonucleic acid (DNA) of bacterial strain HSCY 2073 was used as a template. Polymerase chain reaction (PCR) amplification was carried out by using a universal primer of a bacterial 16S ribosomal ribonucleic acid (rRNA) gene sequence. A 16S rRNA gene sequence of the bacterial strain was obtained, and as shown in SEQ ID NO.1 Blast was carried out in an EzTaxon database (www.ezbiocloud.net). A result showed that homology of 16S rRNA between bacterial strain HSCY 2073 in the present disclosure and bacterial strain *Acetilactobacillus jinshanensis* HSLZ-75 was 99% or above. The bacterial strain in the present disclosure was named *Lactobacillus sp.* HSCY 2073 in combination with physiological and biochemical features and a feature of metabolizing sugar to produce lactic acid of the bacterial strain.

The bacterial strain is deposited in China General Microbiological Culture Collection Center. Deposit place is Institute of Microbiology, Chinese Academy of Sciences, No.3, Yard 1, Beichen West Road, Chaoyang District, Beijing, China. Accession number of deposit is CGMCC NO. 18982. Deposit date is 21 November 2019.

### (6) Comparison of main application features of bacterial strains

A comparative research was carried out on bacterial strain HSCY 2073 in the present disclosure and new lactobacillus species *Acetilactobacillus jinshanensis* HSLZ-75. Results showed that bacterial strain HSCY 2073 could produce lactic acid by reducing sugar, and therefore was extremely suitable for a brewing environment of traditional solid-state vinegar. Maximum tolerated total acid was 8.0 g/100 mL, while bacterial strain HSLZ-75 could tolerate total acid of 6.5 g /100 mL to a maximum extent under the same conditions (see Table 1 for details). Bacterial strain HSCY 2073 could tolerate ethanol of 13%, and could metabolize at 15°C. An optimum growth temperature was 30°C, which was also obviously different from that of bacterial strain HSLZ-75.

Under the same culture conditions, a content of non-volatile acid produced through metabolism of bacterial strain HSCY 2073 in the present disclosure was increased by 7.21% than bacterial strain HSLZ-75. In cases of 5 substances having fruit aromas, a content of acetaldehyde was increased by 3.15%, a content of ethyl acetate was increased by 8.39%, a content of isobutyl acetate was increased by 0.55%, a content of isoamyl acetate was increased by 1.93%, and a content of ethyl 3-methylvalerate was increased by 14.13%. Bacterial strain HSCY 2073 has a stronger application prospect of industrial improved liquid MRS culture medium.

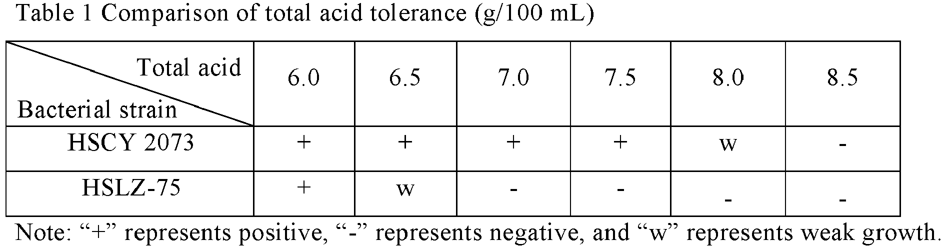

### Example 2

Enhanced application of bacterial strain HSCY 2073 in Zhenjiang aromatic vinegar brewing

The example provides enhanced application by combining bacterial strain HSCY 2073 in the present disclosure and a starter solid-state vinegar culture of traditional Zhenjiang aromatic vinegar brewing.

### (1) Preparation of seed solution

### 1. Activation and propagation of bacterial strains

Bacterial strain HSCY 2073 that was deposited as a slant at 4 °C was inoculated into 10 mL of a sterilized improved liquid MRS culture medium by using an inoculation loop. Total acid of the improved liquid MRS culture medium was adjusted to 5.0 g/100 mL by using acetic acid. Sealing, standing and culturing were carried out at 30°C for 10 days. Then, 5 mL of the above fermentation broth was transferred into 100 mL of the sterilized liquid MRS culture medium. Total acid of the improved liquid MRS culture medium was adjusted to 5.0 g/100 mL by using acetic acid. A number of viable bacteria subjected to sealing and culturing at 30°C was about 10⁷ CFU/mL.

Bacterial strain HSLZ-75 was activated and propagated through the same method.

### 2. Preparation of primary seed solution

A bacterial solution having a number of viable bacteria of about 10⁷ CFU/mL was inoculated into 500 mL of an improved liquid MRS culture medium according to an inoculation amount of 5%(v/v). Total acid of the improved liquid MRS culture medium was adjusted to 5.0 g/100 mL by using acetic acid. Sealing and culturing were carried out at 30°C for 9 days, and a primary seed solution was obtained.

Both bacterial strain HSCY 2073 and bacterial strain HSLZ-75 were propagated through the same method mentioned above.

### 3. Preparation of secondary seed solution

A liquid fermentation cylinder of 15 L was selected. The primary seed solution was inoculated in the improved liquid MRS culture medium according to an inoculation amount of 5%(v/v). Total acid of the improved liquid MRS culture medium was adjusted to 5.0 g/100 mL by using acetic acid. A temperature was kept at 29°C, and pressure maintaining, anaerobic sealing, and culturing were carried out for 8 days. A number of viable bacteria in a final fermentation broth was about 10⁷ CFU/mL, and a secondary seed solution was obtained.

Both bacterial strain HSCY 2073 and bacterial strain HSLZ-75 were propagated through the same method mentioned above.

### (2) Enhanced application in traditional Zhenjiang aromatic vinegar brewing

### 1. Preparation of wine mash

Specifically, 5 large cylinders of 500 kg were provided, and 100 kg of glutinous rice was soaked in water overnight. Glutinous rice was steamed, cold water was poured over rice to about 40°C, 0.6 kg of chinese yeast was added, uniform mixing was carried out, and nesting was carried out in the cylinders to form trumpet shapes. When a certain amount of wine liquid appears in nests, 5.0 kg of wheat koji was added into each cylinder, 300 kg of water was added, and uniform stirring was carried out. In an alcohol fermentation process, stirring was carried out in a non-scheduled manner according to wine brewing requirements, a temperature was controlled at about 30°C, and fermentation was carried out for about 5 days - 7 days until an alcoholic content was not increased any more. After fermentation, wine mash in the 5 large cylinders were uniformly mixed, and an alcohol content was adjusted to 9% vol for later use.

### 2. Grouped feeding and preparation of solid-state vinegar cultures

Test group: 200 kg of the above wine mash was add into each of 3 large cylinders of 400 kg. The secondary seed fermentation broth of bacterial strain HSCY 2073 mentioned above was added. An addition amount was 1.0% of wine mash. 80 kg of bran and 45 kg of chaff were was added. Uniform mixing was carried out. According to a traditional Zhenjiang aromatic vinegar brewing process, 26 kg of a starter solid-state vinegar culture were added to an upper portion of a solid-state vinegar culture in each cylinder, and then covered with chaff for heat preservation.

Control group 1: 3 large cylinders were selected. the secondary seed fermentation broth of bacterial strain HSCY 2073 was replaced with distilled water. Operations were carried out according to the same addition amount and addition method as the above test group.

Control group 2: 3 large cylinders were selected. The secondary seed fermentation broth of bacterial strain HSLZ-75 was cultured through the same method as mentioned above. The secondary seed fermentation broth of bacterial strain HSCY 2073 was replaced with the secondary seed fermentation broth of bacterial strain HSLZ-75. Operations were carried out according to the same addition amount and addition method as the above test group.

### 3. Fermentation and sealing

According to a traditional "solid-state layered fermentation technique" of Zhenjiang aromatic vinegar, solid-state vinegar cultures were turned layer by layer. The solid-state vinegar cultures were fermented until total acid of the solid-state vinegar cultures was not increased. The solid-state vinegar cultures were sealed and compacted. Salt was added. The solid-state vinegar cultures were sealed for 30 days.

### 4. Preparation of finished products

After sealing, a solid-state vinegar culture was added into a vinegar pouring cylinder, and then a parched rice color was added for vinegar pouring. Vinegar frying, aging, sterilization and filling were carried out, and a finished product was obtained.

### (3) Enhanced application effect in traditional Zhenjiang aromatic vinegar brewing as shown in Tables 2-4.

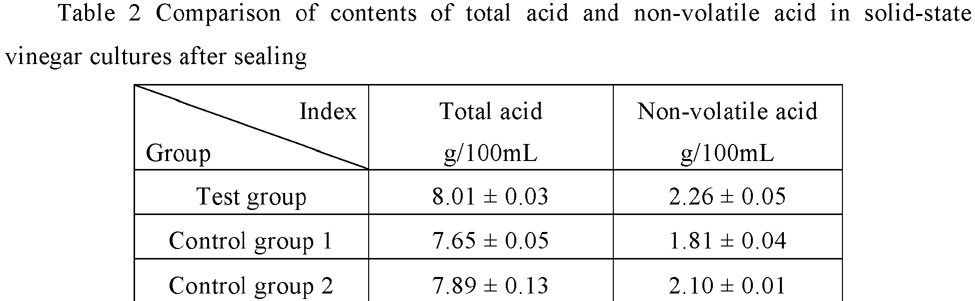

Compared with control group 1, the test group enhanced by the bacterial strain in the present disclosure had effects that in the solid-state vinegar culture after sealing, a content of total acid was increased by 4.71%, a content of non-volatile acid was increased by 24.86%, and a yield of products was increased by 5.14%. Compared with control group 2, after enhancement by the bacterial strain in the present disclosure, in a vinegar liquid, a content of total acid was increased by 1.52%, a content of non-volatile acid was increased by 7.62%, and a yield of products was increased by 1.82%.

**Table 3 Contents of 5 aroma substances in solid-state vinegar cultures after sealing**

| Substance name | Aroma Description | Increased amount % of test group compared with control group 1 | Increased amount % of test group compared with control group 2 |
|---|---|---|---|
| Acetaldehyde | Fruit aroma | 9.05 | 3.67 |
| Ethyl acetate | Fruit aroma | 25.83 | 6.57 |
| Isobutyl acetate | Glue smell, fruit aroma | 1.51 | 0.39 |
| Isoamyl acetate | Fruit aroma | 7.39 | 2.01 |
| Ethyl 3-methylvalerate | Fruit aroma | 34.27 | 15.39 |

Compared with control groups 1 and 2, the test group enhanced by the bacterial strain in the present disclosure had effects that 5 aroma substances having fruit aromas in solid-state vinegar cultures after sealing were all increased. Especially ethyl 3-methylvalerate was increased most highly.

**Table 4 Comparative analysis of sensory quality of finished vinegar**

| Group | Color (10 scores) | Posture (10 scores) | Aroma (10 scores) | Taste (10 scores) |
|---|---|---|---|---|
| Test group | 9.5 | 9.3 | 9.6 | 9.5 |
| Control group 1 | 9.4 | 9.0 | 8.7 | 8.8 |
| Control group 2 | 9.5 | 9.1 | 9.0 | 9.1 |

Then, 30 experienced aromatic vinegar evaluators were selected to conduct sensory evaluation and analysis on 3 products mentioned above. Through three-point test, it was found that 26 of 30 evaluators indicated that there was a great difference between the products of the test group and control group 1, and 20 evaluators indicated that there was a certain difference between the products of the test group and control group 2. Through descriptive analysis, it was indicated that the products of the test group had more prominent aroma, especially fruit aroma, softer taste and lower irritation. Through preference analysis, it was indicated that the product of the test group had stronger preference. Through comprehensive evaluation, scores of 3 products were similar in color and body, and scores of the test group were significantly higher than those of control group in aroma and taste, and an overall sensory score of the test group was higher.

The present disclosure has remarkable effect on improving overall flavor quality of traditional Zhenjiang aromatic vinegar.

### Example 3

### Enhanced application of bacterial strain HSCY 2073 in Shanxi aged vinegar brewing

The example provides enhanced application in Shanxi aged vinegar brewing by combining the bacterial strain in the present disclosure and a solid-state vinegar culture of traditional Shanxi aged vinegar.

### (1) Preparation of seed solution

Propagation was carried out through the same method as that in Example 2.

### (2) Enhanced application in traditional Shanxi aged vinegar brewing

### 1. Preparation of wine mash

Specifically, 9 large cylinders of 500 kg were provided, 100 kg of ground sorghum with only four to six petals were provided, and 50 kg of water was added to moisten materials for 15 h. The sorghum was steamed, 300 kg of water was added, stirring was carried out, cooling was carried out to 30°C, 60 kg of Daqu was carried in, and uniform stirring was carried out. In an alcohol fermentation process, stirring was carried out in a non-scheduled manner according to wine brewing requirements, a temperature was controlled to be about 30°C, and fermentation was carried out for about 9 days - 15 days until an alcoholic content was not increased any more.

### 2. Feeding and preparation of solid-state vinegar cultures

Test group: after alcohol fermentation, the secondary seed fermentation broth of bacterial strain HSCY 2073 in Example 2 was added into each of 3 large cylinders. An addition amount was 0.6% of mass of wine mash. 200 kg of bran and 160 kg of chaff were added. The wine mash and grains were uniformly mixed. According to a traditional Shanxi aged vinegar brewing process, 10 kg of a solid-state vinegar culture was added into each cylinder.

Control group 1: 3 large cylinders were selected. The secondary seed fermentation broth of bacterial strain HSCY 2073 was replaced with distilled water. Operations were carried out according to the same addition amount and addition method as the above test group.

Control group 2: 3 large cylinders were selected. The secondary seed fermentation broth of bacterial strain HSLZ-75 was cultured through the same method as mentioned above. The secondary seed fermentation broth of bacterial strain HSCY 2073 was replaced with the secondary seed fermentation broth of bacterial strain HSLZ-75. Operations were carried out according to the same addition amount and addition method as the above test group.

### 3. Fermentation, fumigation, and preparation of finished products

According to the traditional Shanxi aged vinegar brewing process, solid-state vinegar cultures were turned. The solid-state vinegar culture was fermented until total acid of the solid-state vinegar culture was not increased any more. 10 kg of salt was added to each cylinder. Fumigation, vinegar pouring and decoction were carried out. Finished vinegar was obtained.

### (3) Enhanced application effects in traditional Shanxi aged vinegar brewing as shown in Tables 5-7.

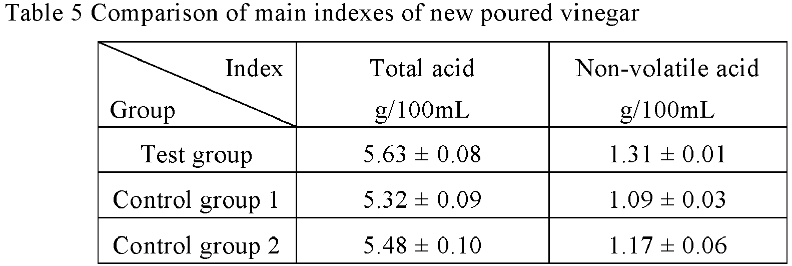

Compared with control group 1, the test group enhanced by the bacterial strain in the present disclosure had effects that a content of total acid in new poured vinegar was increased by 5.83%, and a content of non-volatile acid was increased by 20.18%. Compared with control group 2, after enhancement by the bacterial strain in the present disclosure, a content of total acid in new poured vinegar was increased by 2.74%, and a content of non-volatile acid was increased by 11.97%.

**Table 6 Contents of 5 aroma substances in solid-state vinegar cultures after sealing**

| Substance name | Aroma Description | Increased amount % of test group compared with control group 1 | Increased amount % of test group compared with control group 2 |
|---|---|---|---|
| Acetaldehyde | Fruit aroma | 7.13 | 1.29 |
| Ethyl acetate | Fruit aroma | 19.25 | 4.89 |
| Isobutyl acetate | Glue smell, fruit aroma | 0.89 | 0.44 |
| Isoamyl acetate | Fruit aroma | 5.39 | 2.18 |
| Ethyl 3-methylvalerate | Fruit aroma | 16.79 | 11.35 |

Compared with control groups 1 and 2, the test group enhanced by the bacterial strain in the present disclosure had effects that 5 aroma substances having fruit aromas in solid-state vinegar cultures after sealing were all increased. Especially ethyl acetate was increased most highly.

**Table 7 Comparative analysis of sensory quality of finished vinegar**

| Group | Color (10 scores) | Posture (10 scores) | Aroma (10 scores) | Taste (10 scores) |
|---|---|---|---|---|
| Test group | 9.3 | 9.1 | 9.3 | 9.4 |
| Control group 1 | 9.3 | 9.1 | 8.5 | 8.7 |
| Control group 2 | 9.2 | 9.2 | 9.0 | 9.1 |

Then, 30 experienced aromatic vinegar evaluators were selected to conduct sensory evaluation and analysis on 3 products mentioned above. Through three-point test, it was found that 22 of 30 evaluators indicated that there was a great difference between the products of the test group and control group 1, and 21 evaluators indicated that there was a certain difference between the products of the test group and control group 2. Through descriptive analysis, it was indicated that the product of the test group had more prominent aroma and softer taste. Through preference analysis, it was indicated that the product of the test group had stronger preference. Through comprehensive evaluation, scores of 3 products were similar in color and body, and scores of the test group were significantly higher than those of control group in aroma and taste, and an overall sensory score of the test group was higher.

Although there was a significant difference between Shanxi aged vinegar brewing and Zhenjiang aromatic vinegar brewing in raw material and process, especially in a solid-state vinegar culture environment (nutritional environment, temperature environment, acid, ethanol, etc.), the above examples showed that bacterial strain HSCY 2073 in the present disclosure also has a remarkable effect on improving quality of traditional Shanxi aged vinegar.

**Sequence Table**

| **16S ribosomal ribonucleic acid (rRNA) sequence of bacterial strain HSCY 2073** |
|---|
| AGAGTTTGATCCTGGCTCAGGATGAACGCTGGCGGTGTGCCTAATACATGCAAGT CGCGCGCACTCCCGTAGATGATTTTGATGCTCTGCATCAAATGATTCTACATTCGA GTGAGCGGCGGATGGGTGAGTAACACGTGGGTAACCTGCCCTTGAGTCTGGGATA GCATCTGGAAACGGATAATAATACCGGATAACAACTGAAACCACATGGTTTCAGT TTGAAAGGCGCGTTTGCGTCGCTCTTGGAGGGACCCGCGGTCCATTAGTTAGTTG GTAAGATAAAGGCCTACCAAGACGATGATGGATAGCCGAACTGAGAGGTTGATC GGCCACATTGGAACTGAGACACGGTCCAGACTCCTACGGGAGGCAGCAGTAGGG AATCTTTCACAATGGGCGAAAGCCTGATGAAGCAACACCGCGTGAATGAAGAAG GGTTTCGGCTCGTAAAATTCTGTTGCTAGAGAAGAACGTACCAAGTAGGAAATGG CTTGGTAGTGACGGTATCTGGTTAGAAAGTCACGGCTAACTACGTGCCAGCAGCC GCGGTAATACGTAGGTGGCAAGCGTTATCCGGATTTATTGGGCGTAAAGCGAGCG CAGGCGGTTGTTTAAGTCCGATGTGAAAGCCTTCGGCTTAACCGAAGAAGTGCAT TGGAAACTGAGCGACTTGAGTACAGAAGAGGACAGTAGAACACCATGTGTAGCG GTGAAATGCGTAGATATATGGTAGAATACCAGTGGCGAAAGCGGCTGTCTGGTCT GTCACTGACGCTGAGGCTCGAAAGCATGGGTAGCAAACAGGATTAGATACCCTG GTAGTCCATGCTGTAAACGTTGAGTGCTAGGTGTTAGAGGATTTCCGTCCTTTAGT GCCGAAGTTAACACATTAAGCACTCCGCCTGGGGAGTACGACCGCAAGGTTGAA ACTCAAAGGAATTGACGGGGACCCGCACAAGTGGTGGAGCATGTGGTTTAATTCG ATGCTACGCGAAGAACCTTACCAGGCCTTGACATACTTCTGTTAGGCTAAGAGAT TAGCTGTTCCCTTCGGGGACAGATCTACAGGTGGTGCATGGTCGTCGTCAGCTCG TGTCGTGAGATGTTGGGTCAAGTCCCGCAACGAGCACAACCCTTGTGACTAGTTG CCAGCATTCAGTTGGGCACTCTAGTCAGACTGCCGGTGATAAACCGGAGGAAGGT GAGGATGACGTCTGATCATCATGCCCCTTATGGCCTGGGCTACACACGTGCTACA ATGGACAGTACAATGGGTTGCGAAACCGCGAGGTCAAGCTTATCCCATAAAGCTG TTCTCAGTTCGGACTGCAGTCTGCAACTCGACTGCACGAAGCTGGAATCACTAGT AATCGTGGATCAGCATGCCACGGTGAATACGTTCCCGGGTCTTGTACATACCGCC CGTCACACCATGAGAGTCCGTAACACCCAAAGATAGTGCGGCAACCTCTTTTGAG GAGCCAGCTATCTAAGGTGGGACGAATGATTAGGGTGAAGTCGTAACAACGTAG CCGTA |

## Claims

1. Bacterial strain HSCY 2073, identified as *Lactobacillus sp.* and deposited in China General Microbiological Culture Collection Center, wherein deposit date is 21 November 2019, and deposit number is CGMCC No. 18982.

2. Isolation and screening of bacterial strain HSCY 2073 according to claim 1, comprising steps as follows:
(1) collecting solid-state vinegar cultures after fermentation, and carrying out sealing, standing, enriching and culturing;
(2) diluting an enriched sample, and coating, with the enriched sample, an improved solid de man, rogosa and sharpe (MRS) plate for culture;
(3) selecting a bacterial colony having a fast growth speed, large colony area and fruit aroma, and carrying out streaking and purifying on the improved solid MRS plate through a streaking method; and
(4) inoculating a purified bacterial strain into an improved liquid MRS culture medium, carrying out standing and culturing in a sealed culture box, measuring a yield of non-volatile acid, screening out a bacterial strain having a high yield of non-volatile acid, carrying out repetition, and obtaining bacterial strain HSCY 2073 through screening.

3. Application of bacterial strain HSCY 2073 according to claim 1 in improvement on flavor quality of vinegar.

4. The application according to claim 3, wherein bacterial strain HSCY 2073 increases contents of non-volatile acid and aroma substances in the vinegar.

5. The application according to claim 3, wherein bacterial strain HSCY 2073 increases contents of flavor substances including non-volatile acid, acetaldehyde, ethyl acetate, isobutyl acetate, isoamyl acetate and ethyl 3-methylvalerate in the vinegar.

6. Application of bacterial strain HSCY 2073 according to claim 1 in aromatic vinegar brewing.

7. The application according to claim 6, wherein bacterial strain HSCY 2073 and a starter solid-state vinegar culture of aromatic vinegar are combined for enhanced application in aromatic vinegar brewing.

8. Enhanced application of bacterial strain HSCY 2073 according to claim 1 in aged vinegar brewing.

9. The application according to claim 8, wherein bacterial strain HSCY 2073 and a solid-state vinegar culture of aged vinegar are combined for enhanced application in aged vinegar brewing.
